# DEMANDE DE BREVET EUROPEEN

(11) **EP 2 290 093 A2**
(43) Date de publication de la demande: **02.03.2011**
(21) Numéro de dépôt: 10180910.1
(22) Date de dépôt: 19.01.2005
(51) Int. Cl.: C12Q 1/37

(54) **Procédé de détection de la PRP utilisant une molécule ayant au moins une charge positive et/ou au moins une liaison osidique et un ligand autre qu'un ligand proteique**

(30) Priorité: 20.01.2004 FR 0400492; 17.06.2004 FR 0406538
(62) Demande divisionnaire de: 05717449.2
(71) Demandeur: BIOMERIEUX, 69280 Marcy-L'Etoile (FR); Universite Claude Bernard Lyon 1 (UCBL), 69622 Villeurbanne Cedex (FR); Agence Française de Securité Sanitaire des Aliments, 94701 Maisons-Alfort Cedex (FR); CNRS, 75794 Paris Cedex 16 (FR)
(72) Inventeur: Benscik-Reynier, Anna, 38110, Saint Clair de la Tour (FR); Coleman, Anthony William, 69300 Caluire et Cuire (FR); Da Silva, Eric, 69007, Lyon (FR); Dupin, Marilyne, 69670, Vaugneray (FR); Leclere, Edwige, 69001, Lyon (FR); Martin, Ambroise, 69390, Charly (FR); Moussa, Aly, 69600, Oullins (FR); Perron, Hervé, 69290, Saint Genis les Ollieres (FR); Ronzon, Frédéric, 69610, Montromant (FR)
(74) Mandataire: Pascual, Lise Jeanine Solange

(57) **Abrégé**

La présente invention concerne un procédé de détection de la PrP dans un échantillon biologique d'origine humaine ou animale susceptible de contenir de ladite PrP, **caractérisé en ce qu**'il met en oeuvre une molécule ayant au moins une charge positive et/ou au moins une liaison osidique et un ligand autre qu'un ligand protéique choisi parmi les ligands macrocycliques et les glycosaminoglycanes.

## Description

La présente invention concerne le domaine des maladies à prion, et en particulier un procédé de détection des formes du prion liées à ces maladies.

La protéine prion native ou normale, désignée PrP^{c} pour la protéine prion cellulaire, est une glycoprotéine largement exprimée dans les cellules lymphoides et neuronales des mammifères.

Des changements conformationnels de PrP^{c} conduisent à l'apparition et à la propagation de la protéine pathologique PrP^{sc} qui est résistante à la protéinase K. Cette protéine pathologique sera appelée dans la présente demande indifféremment PrP^{sc} ou PrP^{res}. Le terme PrP désignera dans la présente demande toute forme de PrP normale ou non, résistante ou non.

L'accumulation de PrP^{sc} dans les organes des mammifères est à l'origine de nombreuses maladies, appelées maladies à prions ou encéphalopathies spongiformes transmissibles (EST), et notamment de la tremblante des petits ruminants, de la maladie cachectisante chronique (ou chronic wasting disease « CWD ») de l'élan et de l'antilope, de l'encéphalopathie spongiforme bovine (ESB), et de la maladie de Creutzfeld-Jakob (MCJ) chez l'homme.

Le développement de la maladie chez l'hôte infecté se traduit par une accumulation de la protéine pathologique PrP^{sc} dans le cerveau, ce qui conduit à une altération irréversible des cellules du cerveau.

L'apparition tardive après une période d'incubation de 2 à 6 ans et le lent développement des symptômes chez le bétail infecté par l'ESB a considérablement ralenti le développement de modèles épidémiologiques. L'ESB est transmissible par ingestion à l'homme et a conduit à l'apparition d'une nouvelle forme de la maladie de Creutzfeldt-Jakob (vMCJ) chez l'homme.

La détection de la protéine pathologique PrP^{sc} est difficile chez les animaux infectés asymptomatiques avant le développement de la maladie et surtout dans les liquides physiologiques, tels que le sang et l'urine, chez les animaux malades. Il est à présent établi que la PrP^{sc} présente chez les animaux destinés à l'alimentation humaine se transmet à l'homme lors de l'ingestion des tissus infectés. Un objectif majeur de santé publique est donc d'éviter cette transmission en détectant la présence de la PrP^{sc} :
- chez des animaux destinés à la consommation humaine en vue de les retirer de la chaîne alimentaire,
- dans les dons de sang et dérivés sanguins destinés à la transfusion chez l'homme. En effet, comme le montre une présence de protéine pathogène PrP^{sc} dans le sang et les cellules lymphoïdes bien avant l'atteinte cérébrale, et donc bien avant la possibilité de déceler des signes neurologiques évocateurs de maladie à prions cliniquement déclarée, la physiopathologie chez l'homme est méconnue. Ne pouvant réaliser des infections expérimentales comme chez le mouton, l'absence de test de détection dans le sang ou d'autres fluides biologiques ne permet pas de l'étudier et donc de prévenir une transmission interhumaine par don de sang, ou d'envisager un traitement des personnes infectées avant que les lésions cérébrales n'aient débuté ;
- dans les troupeaux animaux avant le stade neurologique, permettant ainsi d'éliminer les animaux infectés précocement, avant arrivée aux abattoirs.

La détection de la présence de PrP^{sc} dans des échantillons biologiques chez l'homme ou chez des animaux devient donc d'une extrême importance et plusieurs équipes de chercheurs développent des méthodes de détections immunologiques (WO02/086511) Par ailleurs, des méthodes pour complexer à la PrP^{sc} des peptides, des petites molécules ou des inhibiteurs en vue du traitement de vMCJ font l'objet de recherches actives. Toutefois, les méthodes de l'état de la technique se heurtent sans arrêt à la difficulté d'identifier la PrP^{sc} de manière fiable lorsqu'elle est en faible quantité dans un échantillon biologique.

Les présents inventeurs ont maintenant mis en évidence contre toute attente que l'utilisation d'une molécule ayant au moins une charge positive et/ou au moins une liaison osidique et d'un ligand autre qu'un ligand protéique dans un test pour le diagnostic de la PrP dans un échantillon biologique susceptible de contenir une telle protéine, permettait la détection de cette protéine à des dilutions et dans des conditions où elle n'est pas détectable avec les méthodes utilisées actuellement, l'utilisation de ces deux composants en combinaison ne modifiant pas la capacité de liaison de la PrP à un partenaire de liaison spécifique de la PrP utilisé dans le test diagnostic.

Ainsi, la présente invention a pour objet un procédé de détection de la PrP, notamment PrP^{sc}, dans un échantillon biologique d'origine humaine ou animale susceptible de contenir de la PrP, **caractérisé en ce qu**'il met en oeuvre une molécule ayant au moins une charge positive et/ou au moins une liaison osidique et un ligand autre qu'un ligand protéique.

Elle concerne également les kits de diagnostic pour la détection de la PrP, comprenant un ligand autre qu'un ligand protéique et une molécule ayant au moins une charge positive et/ou au moins une liaison osidique.

Par ligand autre qu'un ligand protéique, on entend un composé capable de se lier à la PrP et de nature non protéique. A titre de ligand protéique exclu de cette définition de ligand, on peut citer les anticorps anti-PrP.

Par molécule ayant au moins une charge positive et/ou au moins une liaison osidique on entend une molécule ayant au moins une charge positive ou une molécule ayant une fonction engagée dans une liaison osidique, ou bien les deux.

Le procédé de l'invention a donc pour avantage de permettre la détection de la PrP dans des échantillons biologiques où elle est présente en faible quantité grâce à l'utilisation combinée d'une molécule ayant au moins une charge positive et/ou au moins une liaison osidique et d'un ligand autre qu'un ligand protéique tel que défini ci-dessus.

Les échantillons biologiques dans lesquels le procédé de détection de l'invention est mis en oeuvre sont tout échantillon d'origine animale ou humaine susceptible de contenir de la PrP.

A titre d'exemple de tels échantillons, on peut citer le cerveau, les tissus du système nerveux central, les organes tels que la rate et l'intestin, ainsi que les fluides biologiques tels que le liquide céphalo-rachidien, l'urine et le sang.

Les animaux qui peuvent être concernés par le procédé de l'invention sont les animaux qui développent les maladies à prion. On citera à titre d'exemple non limitatif les ovins et les bovins.

A titre de charge positive contenue dans les molécules ayant au moins une charge positive et/ou au moins une liaison osidique, on préfère les charges positives apportées par des fonctions basiques, telles que des fonctions guanidinium ou ammonium.

A titre de liaison osidique contenue dans les molécules ayant au moins une charge positive et/ou au moins une liaison osidique, on préfère les liaisons de type hétéroside telles que celles retrouvées dans les macrolides et les aminoglycosides.

Les molécules ayant au moins une charge positive et/ou au moins une liaison osidique préférées aux fins de l'invention sont les molécules ayant à la fois au moins une charge positive et au moins une liaison osidique, ainsi que les molécules ayant au moins deux fonctions guanidinium et/ou ammonium, de préférence dans un système moléculaire hydrophile non polymérique.

Selon un mode de réalisation particulier de l'invention, les molécules ayant au moins une charge positive et au moins une liaison osidique sont choisies parmi les aminoglycosides à noyau guanidinium, la streptomycine étant particulièrement préférée.

Selon un autre mode de réalisation particulier de l'invention, les molécules ayant au moins deux fonctions guanidinium et/ou ammonium sont choisies parmi la polyallylamine, le triéthylènetétraamine (TET), la bis-3-aminopropylamine, le tétrachlorhydrate de spermine, le sesquisulfate de dihydrostreptomycine, la streptomycine, ces deux derniers composés appartenant également à la famille des molécules ayant au moins une charge positive et au moins une liaison osidique.

L'utilisation d'une molécule ayant au moins une charge positive et/ou au moins une liaison osidique, en particulier d'une molécule ayant au moins deux fonctions guanidinium et/ou ammonium, de préférence la streptomycine, de préférence encore sous forme de sel, permet la précipitation de la PrP présente dans l'échantillon biologique testé. Cette propriété de précipitation est accrue en présence de la PrP^{sc}, en particulier après traitement à la protéinase K (PrP^{res}). Cette précipitation est due à la formation d'agrégats de PrP (également appelés PrP réticulée par la molécule ayant au moins une charge positive et/ou au moins une liaison osidique) obtenus après traitement par la molécule ayant au moins une charge positive et/ou au moins une liaison osidique.

Ainsi, il est possible de détecter de la PrP, en particulier de la PrP^{sc}, par un procédé caractérisé par le fait que l'on met en présence un échantillon biologique issu ou obtenu d'un organisme animal ou humain avec une molécule ayant au moins deux fonctions guanidinium et/ou ammonium, à l'exception des antibiotiques répondant à cette définition, tels que la streptomycine.

Ce procédé peut être mis en oeuvre par les étapes consistant à :
a) ajouter, à l'échantillon biologique issu ou obtenu d'un organisme animal ou humain, une molécule ayant au moins deux fonctions guanidinium et/ou ammonium, à l'exception des antibiotiques,
b) soumettre la solution à un chauffage (par exemple entre 37 et 150°C), puis à une centrifugation et séparer le culot du surnageant,
c) détecter la PrP^{sc} après migration sur un gel d'électrophorèse, transfert et immunodétection ou après capture sur support solide suivie d'une immunodétection de type ELISA.

Le procédé peut également comprendre comme étape supplémentaire une étape de dénaturation dans les conditions indiquées ci-après.

Le procédé peut également comprendre comme étape supplémentaire, de préférence précédant l'étape a), une étape d'addition de protéinase K dans les conditions indiquées ci-après.

De même l'étape de détection de la PrP^{sc} peut être mise en oeuvre dans les conditions indiquées ci-après.

Du fait de leur capacité à se lier à la PrP^{sc}, les molécules ayant au moins deux fonctions guanidinium et/ou ammonium, à l'exception des antibiotiques, sont particulièrement utiles pour la précipitation, la détection et/ou le diagnostic de la PrP^{sc} même lors de la détection en immunohistochimie, ainsi que pour l'élimination de la PrP^{sc} d'un tissu ou d'un fluide biologique.

L'ajout d'un ligand autre qu'un ligand protéique, et en particulier d'un ligand macrocyclique ou d'un glycosaminoglycane, permet, à la différence des ligands protéiques, d'amplifier la sensibilité de détection de la protéine PrP et de mieux capturer la PrP réticulée par la molécule ayant au moins une charge positive et/ou au moins une liaison osidique. De ce fait, la PrP peut être détectée dans des échantillons biologiques où elle n'est présente qu'en de faibles quantités.

La quantité de molécule ayant au moins une charge positive et/ou au moins une liaison osidique, ainsi que du ligand autre qu'un ligand protéique, peut être facilement déterminée par l'homme du métier en fonction des spécificités de l'échantillon. Ainsi, par exemple, la quantité de molécule ayant au moins une charge positive et/ou au moins une liaison osidique, telle que la streptomycine, peut être comprise entre 50 et 500 mg/ml, de préférence entre 100 et 300 mg/ml.

Contre toute attente, l'action d'une molécule ayant au moins une charge positive et/ou au moins une liaison osidique sur la PrP, puis la complexation du ligand autre qu'un ligand protéique à l'agrégat de PrP ainsi formé et vice-versa, ne gêne en rien la détection de la PrP, par exemple à l'aide d'un anticorps de détection anti-PrP.

Selon un mode de réalisation particulier de l'invention, ladite molécule ayant au moins une charge positive et/ou au moins une liaison osidique est ajoutée audit échantillon biologique pour la précipitation de la PrP avant l'ajout du ligand autre qu'un ligand protéique.

De préférence, pour favoriser la précipitation de la PrP, après ajout de la molécule ayant au moins une charge positive et/ou au moins une liaison osidique, on procède à un chauffage modéré du milieu réactionnel à une température comprise entre 25 et 45°C, une température de 37°C étant préférée.

Avant mise en contact de l'échantillon biologique à tester avec ladite molécule ayant au moins une charge positive et/ou au moins une liaison osidique, l'échantillon peut être préalablement traité avec de la protéinase K pour permettre la digestion protéolytique de la PrP cellulaire. L'échantillon ainsi traité ne contient plus, comme protéine prion, que la protéine PrP^{sc} résistante (PrP^{res}). L'étape de traitement à la protéinase K est une étape discriminante pour la détermination des maladies à prions ou de la contamination par la PrP^{sc} des échantillons testés en l'absence d'anticorps ou ligand spécifique de la PrP^{sc} ou de la PrP^{res}.

La quantité de protéinase K à utiliser dans le procédé de l'invention peut être facilement déterminée par l'homme du métier. Ainsi, par exemple, elle peut être comprise entre 80 et 160 µg/ml.

Ainsi, selon un mode de réalisation préféré, le procédé de détection de la PrP de l'invention comprend l'étape supplémentaire d'ajout de protéinase K dans l'échantillon. Cette étape de digestion par la protéinase K peut être réalisée soit avant réticulation de la PrP avec ladite molécule ayant au moins une charge positive et/ou au moins une liaison osidique, soit après une telle réticulation.

Selon encore un autre mode, le procédé de l'invention comprend les étapes consistant à :
a) ajouter audit échantillon de la protéinase K pour digérer la PrP^{c},
b) ajouter au mélange ainsi obtenu ladite molécule ayant au moins une charge positive et/ou au moins une liaison osidique pour obtenir des agrégats de PrP,
c) ajouter un ligand autre qu'un ligand protéique et
d) révéler la présence de PrP^{res}.

Les agrégats de PrP, formés en présence de la molécule ayant au moins une charge positive et/ou au moins une liaison osidique et contenant la PrP, peuvent être séparés du milieu réactionnel avant leur réaction avec le ligand autre qu'un ligand protéique, qu'il y ait ou non traitement préalable avec la protéinase K. Le procédé de séparation est mis en oeuvre par tout procédé de séparation d'un précipité connu de l'homme du métier. A titre d'exemple, les agrégats de PrP sont séparés du milieu réactionnel par centrifugation, puis par élimination du surnageant. Cette étape de séparation permet d'éliminer tous produits non nécessaires à la réaction de détection ultérieure de la PrP tels que la protéinase K le cas échéant, les protéines digérées et la molécule ayant au moins une charge positive et/ou au moins une liaison osidique libre en solution.

Pour améliorer davantage la sensibilité du procédé de détection de l'invention, avant la réaction des agrégats de PrP avec le ligand autre qu'un ligand protéique, on peut procéder également à une dénaturation desdits agrégats présents dans l'échantillon biologique à tester. Cette étape de dénaturation peut être mise en oeuvre par tout procédé de dénaturation d'agrégats protéiques connu de l'homme du métier. De préférence, la dénaturation est mise en oeuvre par ajout de guanidine HCl.

Ainsi, le procédé de détection de la PrP selon l'invention comprend de préférence au moins l'une des étapes supplémentaires i) et ii) suivantes consistant à :
i) séparer les agrégats de PrP du mélange réactionnel et
ii) dénaturer les agrégats de PrP.
ces étapes étant incluses le cas échéant entre l'étape b) et l'étape c).

Selon un mode de réalisation préféré, le procédé de détection de l'invention met en oeuvre les deux étapes i) et ii) successivement, de préférence entre l'étape b) et l'étape c).

La révélation de la présence de la PrP dans un échantillon biologique selon le procédé de l'invention peut être mise en oeuvre selon les procédés classiques de détection d'analytes dans un échantillon.

Elle peut par exemple être mise en oeuvre par une détection immunologique ou non.

Par détection immunologique, on entend la mise en évidence d'une réaction immunologique avec la PrP, cette réaction immunologique consistant en la liaison entre la PrP à détecter et un partenaire de liaison spécifique de la PrP ou bien en une réaction de compétition entre la PrP susceptible d'être contenue dans l'échantillon à tester et de la PrP marquée.

A titre de détection non immunologique, on peut citer par exemple les techniques de coloration sur gel d'électrophorèse bien connues de l'homme du métier.

La détection de la PrP par réaction immunologique peut-être mise en oeuvre par exemple après addition d'un partenaire de liaison spécifique de la PrP.

Par partenaire de liaison spécifique de la PrP, on entend tout partenaire capable de se lier à la PrP. La visualisation de la réaction immunologique consistera alors en la visualisation du complexe partenaire de liaison spécifique de la PrP/PrP.

Selon un mode de réalisation préféré, le procédé de l'invention est tel qu'on ajoute un partenaire de liaison spécifique de la PrP pour la réaction immunologique entre le partenaire de liaison spécifique de la PrP et la PrP, le cas échéant dans l'étape d).

A titre de partenaire de liaison spécifique de la PrP, on peut citer, par exemple, les anticorps, les fragments d'anticorps, les polypeptides, les protéines, les acides nucléiques, les haptènes et les aptamères.

Le terme « anticorps » inclut les anticorps polyclonaux ou monoclonaux, les anticorps obtenus par recombinaisons génétiques et des fragments d'anticorps.

Les anticorps polyclonaux peuvent être obtenus par immunisation d'un animal avec au moins un antigène cible d'intérêt, dans le cas présent la PrP, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment la PrP.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un antigène cible d'intérêt, le cas présent la PrP, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

A titre d'anticorps appropriés pour l'invention, on peut citer par exemple les anticorps 8G8, 12F10 (SpiBio, France) et 3F4 (Immunok).

Les fragments d'anticorps sont tels qu'ils conservent la fonction de liaison la PrP.

Par « polypeptide », on entends un enchaînement d'au moins deux acides aminés. Par acides aminés, on entend les acides aminés primaires qui codent pour les protéines, les acides aminés dérivés après action enzymatique comme la *trans*-4-hydroxyproline et les acides aminés naturels, mais non présents dans les protéines comme la norvaline, la N-méthyl-L-leucine, la staline (Hunt S. dans Chemistry and Biochemistry of the amino acids, Barett GC, ed., Chapman and Hall, London, 1985), les acides aminés protégés par des fonctions chimiques utilisables en synthèse sur support solide ou en phase liquide et les acides aminés non naturels.

Le terme « protéine » inclut les holoprotéines et les hétéroprotéines comme les nucléoprotéines, les lipoprotéines, les phosphoprotéines, les métalloprotéines et les glycoprotéines aussi bien fibreuses que globulaires.

Par acide nucléique, on entend les oligonucléotides, les acides désoxyribonucléiques et les acides ribonucléiques, ainsi que leurs dérivés.

Le terme « oligonucléotide » désigne un enchaînement d'au moins 2 nucléotides (désoxyribonucléotides ou ribonucléotides, ou les deux), naturels ou modifiés. Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine. Pour illustrer une liaison internucléotidique modifiée, on peut mentionner les liaisons phosphorothioate, N-alkylphosphoramidate, alkylphosphonate et alkylphosphodiester. Les alpha-oligonucléotides tels que ceux décrits dans FR-A-2 607 507, les LNA tels que phosphorothioate-LNA and 2'-thio-LNA décrits dans Bioorganic & Medicinal Chemistry Letters, Volume 8, Issue 16, 18 August 1998 ,pages 2219-2222, et les PNA qui font l'objet de l'article de M. Egholm et al., J. Am. Chem. Soc. (1992), 114, 1895-1897, sont des exemples d'oligonucléotides constitués de nucléotides dont le squelette est modifié.

Le terme « haptène » désigne des composés non immunogènes, c'est-à-dire incapables par eux-mêmes de promouvoir une réaction immunitaire par production d'anticorps, mais capables d'êtres reconnus par des anticorps obtenus par immunisation d'animaux dans des conditions connues, en particulier par immunisation avec un conjugué haptène-protéine. Ces composés ont généralement une masse moléculaire inférieure à 3000 Da, et le plus souvent inférieure à 2000 Da, et peuvent être par exemple des peptides glycosylés, des métabolites, des vitamines, des hormones, des prostaglandines, des toxines ou divers médicaments, les nucléosides et les nucléotides.

Les aptamères sont des partenaires de capture de nature protéique et nucléique qui ont pour fonction d'agir en tant qu'anticorps et de se lier à des ligands protéiques (Toulmé, J.J. et Giege, R. , 1998, Medecine Science, 14(2), 155-166).

Ces polypeptides, protéines, haptènes et aptamères ont tous la capacité de se lier à la PrP ou à l'agrégat de PrP.

La visualisation de la réaction immunologique entre le partenaire de liaison spécifique de la PrP et la PrP mise en oeuvre, notamment dans l'étape d), peut être effectuée par tout moyen de détection connu de l'homme du métier, tels que des moyens directs ou indirects.

Dans le cas de la détection directe, c'est-à-dire sans l'intermédiaire d'un marquage, on observe la réaction immunologique par exemple par résonance plasmon ou par voltamétrie cyclique sur une électrode portant un polymère conducteur.

Dans le cas de la détection indirecte, c'est-à-dire par l'intermédiaire d'un marquage, le marquage peut être effectué par l'intermédiaire dudit partenaire de liaison spécifique de la PrP qui sera alors préalablement marqué.

La visualisation de la présence de la PrP dans un échantillon biologique selon le procédé de l'invention peut également être mise en oeuvre selon une méthode dite de compétition. On ajoute alors, notamment dans l'étape d), à la place du partenaire spécifique de liaison de la PrP, de la PrP préalablement marquée. Dans ce cas, le signal de détection est maximal en l'absence de PrP, puis diminue progressivement à mesure que la concentration en PrP recherchée, non marquée, augmente par la réaction de compétition.

Par marquage, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés, luminescents, colorants,
- les molécules radioactives comme le ³²P, le ³⁵S ou le ¹²⁵I,
- les molécules fluorescentes telles que la fluorescéine, la rhodamine, l'alexa ou les phycocyanines, et
- les particules telles que les particules en or, en latex magnétique, les liposomes.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter à l'article J. Histochem. Cytochem. 45 : 481-491, 1997.

Le marquage de protéines est largement connu de l'homme du métier et est décrit par exemple par Greg T. Hermanson dans Bioconjugate Techniques, 1996, Academic Press Inc, 525B Street, San Diego, CA92101 USA.

Selon le type de marquage utilisé, comme par exemple en utilisant une enzyme, l'homme du métier ajoutera des réactifs permettant la visualisation du marquage.

De tels réactifs sont largement connus de l'homme du métier et sont décrits notamment dans Principles and Practice of Immunoessay, 2^{nd} Edition, Edited by C. Price, D.J. Newman Stockton Press, 1997, 345 Park Avenue South, New York.

La détection de la PrP peut être une détection en phase solide, c'est-à-dire utilisant une phase solide sur laquelle est immobilisé un partenaire de capture destiné à la capture de la protéine à détecter. Dans le cas de la présente invention, c'est le ligand autre qu'un ligand protéique qui sert de partenaire de capture préalablement immobilisé sur un support solide. Un exemple de détection en phase solide bien connu de l'homme du métier est la détection de type sandwich, telle que la détection de type ELISA.

Ainsi, selon un mode de réalisation préféré de l'invention, le ligand autre qu'un ligand protéique est lié à un support solide.

A titre de support solide, on peut citer par exemple les billes, telles que les billes magnétiques, et les plaques de microtitrage.

Le ligand autre qu'un ligand protéique peut être lié au support solide de façon connue de l'homme du métier, telle que par adsorption ou liaison covalente, la liaison covalente étant préférée.

Ainsi, le support solide peut être fonctionnalisé par une fonction susceptible de former une liaison avec une fonction portée par le ligand. Suivant un mode de réalisation préféré, le support solide est fonctionnalisé par une liaison NHS (N-hydroxysuccinimide) ou par une fonction NH₂. Cette fonction peut réagir avec une fonction portée sur le ligand. Dans ce mode de réalisation, les ligands portant une fonction susceptible de réagir pour former une liaison avec la liaison fonctionnelle du support solide, notamment portant une liaison NH₂ ou COOH, sont particulièrement préférés.

Des exemples de ligands autre que des ligands protéiques comprennent par exemple les ligands macrocycliques et les glycosaminoglycanes.

Ces ligands ont tous la particularité, à la différence des ligands protéiques, d'amplifier la sensibilité de détection de la protéine PrP.

Selon un mode de réalisation de l'invention, le ligand autre qu'un ligand protéique est choisi parmi les ligands macrocycliques et les glycosaminoglycanes.

Les glycosaminoglycanes sont largement connus de l'homme du métier et sont décrits par exemple dans Polysaccharides, M. Yalpani, Elsevier, Amsterdam, 1988.

A titre de glycosaminoglycanes appropriés aux fins de l'invention, on peut citer par exemple l'héparine, le sulfate de chondroïtine, le sulfate de dermatane, l'acide hyaluronique et le sulfate de kératane.

Par ligand macrocyclique, on entend un composé constitué d'une succession de cycles formant un macrocycle.

Les ligands macrocycliques sont connus de l'homme du métier. A titre d'exemples non limitatifs, on peut citer les cyclophanes, les métacyclophanes, les cyclodextrines, les cyclo(tétra-acides chromotropiques), les sphérands et les cyclo[n]vératrylènes.

Les ligands macrocycliques ont pour avantage particulier qu'ils permettent de piéger la protéine à tester, sous forme libre ou sous forme d'agrégat, par un effet cage.

Les ligands macrocycliques peuvent être préparés selon les techniques connues de l'homme du métier, par exemple décrites dans Comprehensive Supramolecular Chemistry, Pergamon, Oxford, 1996.

Les ligands macrocycliques préférés pour le procédé de l'invention sont choisis parmi les métacyclophanes, les calix-arènes étant particulièrement préférés. De tels composés calix-arènes peuvent être obtenus suivant la méthodologie décrite dans Arduini, A. et al., 1996, Macrocycle Synthesis, Eds. Harwood, L.M. & Moddy,C.J. Oxford University Press, Oxford et Da Silva et al., 2001, J. Supramol. Chem., 1 :135-138.

Selon un mode de réalisation préféré, le ligand macrocyclique de l'invention répond à la formule générale (I) suivante : 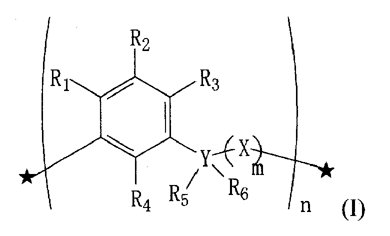 dans laquelle
R₁ représente un atome d'hydrogène, un groupe hydroxyle, un groupe OR ou un groupe OCOR, R étant tel que défini ci-dessous,
R₂ représente un atome d'hydrogène, un groupe R, COR, Pol, CH₂Pol, dans lesquels Pol représente un groupe phosphate, sulfate, amine, ammonium, acide carboxylique et R est tel que défini ci-dessous,
R₃ représente un atome d'hydrogène, un groupe hydroxyle, un groupe OR ou un groupe OCOR dans lesquels R est tel que défini ci-dessous,
R₄ représente un atome d'hydrogène, un groupe hydroxyle, un groupe OR, un groupe OCH₂R ou un groupe OCOR, dans lesquels R est tel que défini ci-dessous,
Y est un atome de carbone, d'azote, ou un atome de soufre,
R₅ et R₆, chacun indépendamment, sont absents ou représentent un atome d'hydrogène, un groupement CH₂ ou R tel que défini ci-dessous, ou bien
R₅ et R₆ représentent ensemble un atome d'oxygène ou de soufre,
X représente un groupement CH₂, ou un atome d'oxygène ou de soufre,
m représente un entier égal à 0 ou 1,
R représente un atome d'hydrogène ou une chaîne hydrocarbonée, saturée ou non, ramifiée ou non, cyclique ou non cyclique, substituée ou non par un groupement halogène, et portant des fonctions polaires ou non polaires,
n est un entier compris entre 3 et 15,
les substituants R₁ à R₅, R, X, Y et l'entier m peuvent être de nature différente suivant les motifs.

Ainsi, le composé de formule (I) se présente sous forme d'une succession de n motifs caractérisés par la présence d'un cycle benzénique, et les substituants de ce cycle peuvent être variables d'un motif à l'autre, dans la limite de leurs définitions ci-dessus.

Les chaînes hydrocarbonées, saturées ou non, ramifiées ou non, cycliques ou non cycliques, substituées ou non par un groupement halogène, et portant des fonctions polaires ou non polaires, sont largement connues de l'homme du métier. A titre d'exemples, on peut citer les alkyles, les alcènes, les aryles et les cycles saturés tels que le cyclohexane. Un exemple de groupement non polaire est CF₃ et des exemples de groupements polaires sont les substituants Pol tels que définis précédemment.

Les composés de formule (I) particulièrement préférés répondent à la formule (Ia) suivante : 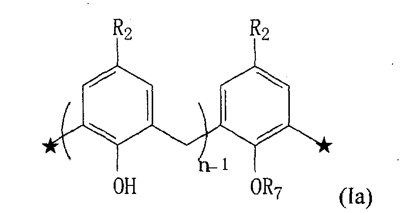 dans laquelle
n est un entier compris entre 4 et 8,
chaque groupement R₂, pris indépendamment, est un groupement sulfate ou un groupement phosphate
R₇ représente un groupement (CH₂)ₜ-(CO)ₛ-(NH₂) ou un groupement (CH₂)ₜ-COOH où t est un entier compris entre 0 et 6 et s est un entier compris entre 0 et 6.

Les composés de formule (Ia) particulièrement préférés sont ceux pour lesquels les deux groupements R₂ sont chacun un groupement sulfate, n est 4, 6 ou 8, et R₇ est un atome d'hydrogène, un groupement -CH₂COOH, un groupement -CH₂CONH₂ ou un groupement -CH₂CH₂NH₂, ce qui constitue un mode de réalisation de l'invention.

Selon un mode de réalisation préféré, le ligand macrocyclique répond à la formule générale (Ia) dans laquelle n=6, X=Y=sulfate et R₇ est -CH₂CH₂NH₂.

Pour la mise en oeuvre du procédé de détection immunologique de la PrP pathogène de l'invention, on peut utiliser des kits de diagnostic comprenant un ligand autre qu'un ligand protéique, de préférence un ligand macrocyclique, et une molécule ayant au moins une charge positive et/ou au moins une liaison osidique.

Selon un mode de réalisation préféré, ledit ligand autre qu'un ligand protéique présent dans le kit est lié à un support solide pour la mise en oeuvre de la détection de la PrP pathogène selon un procédé de détection en phase solide.

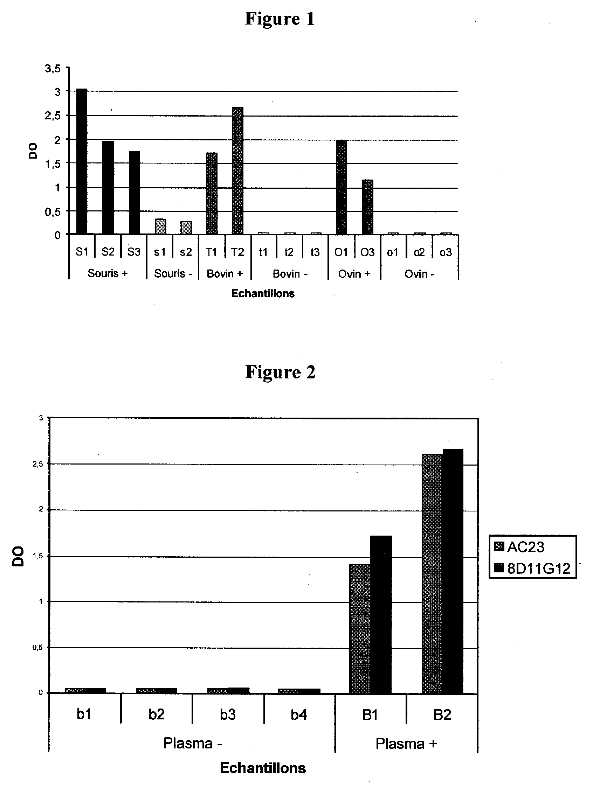

L'invention sera mieux comprise à l'aide des exemples suivants donnés à titre illustratif et non limitatif, ainsi qu'à l'aide des figures 1 à 10, dans lesquelles :
- la figure 1 est une représentation graphique donnant les valeurs de DO obtenues après détection par le procédé de l'invention de PrP^{res} dans des échantillons de sérum de souris, de sérums d'ovins et de plasmas de bovins, positifs (+) ou négatifs (-),
- la figure 2 est une représentation graphique donnant les valeurs de DO obtenues après détection par le procédé de l'invention de PrP^{res} dans des échantillons de plasmas de bovins positifs (plasma +) ou négatifs (plasma -) en utilisant deux anticorps de révélation différents,

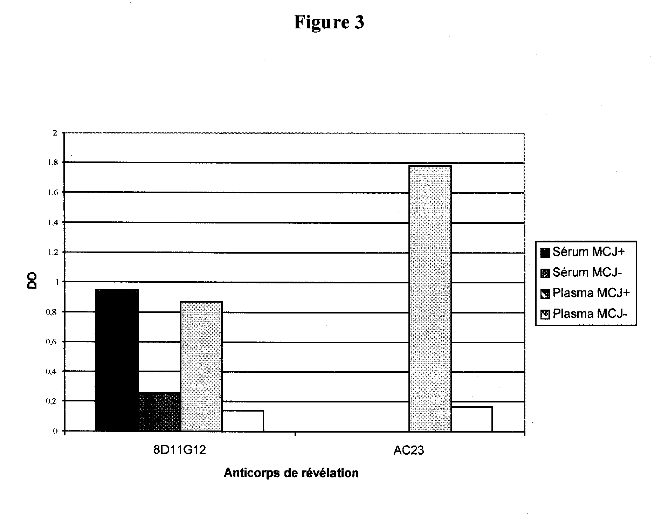

- la figure 3 est une représentation graphique donnant les valeurs de DO obtenues après détection par le procédé de l'invention de PrP^{res} dans des échantillons de sérums et de plasmas humains positifs vis-à-vis de la maladie de Creutzfeld-Jakob (MCJ +) ou négatifs (MCJ -) en utilisant deux anticorps de révélation différents,

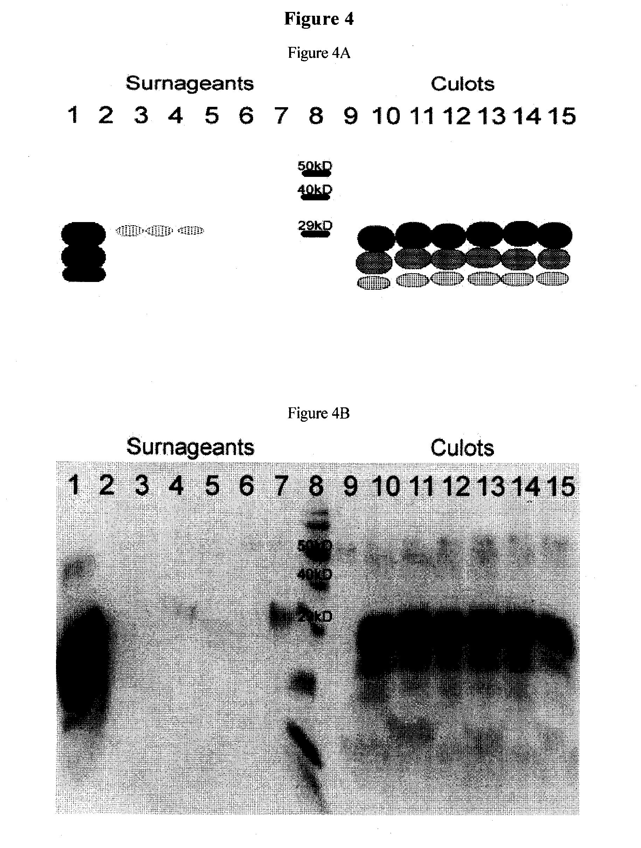

- la figure 4 est une représentation schématique (figure 4A) d'un gel d'électrophorèse (figure 4B) à la suite d'un Western Blot, obtenu après migration d'échantillons de cerveau bovin de BSE traités soit par du sesquisulfate de streptomycine (pistes 5-7 et 13-15), soit par de la streptomycine (pistes 2-4 et 10-12), les pistes 1 et 9 correspondant au témoin non traité et la piste 8 correspondant à la piste des marqueurs de poids moléculaire,

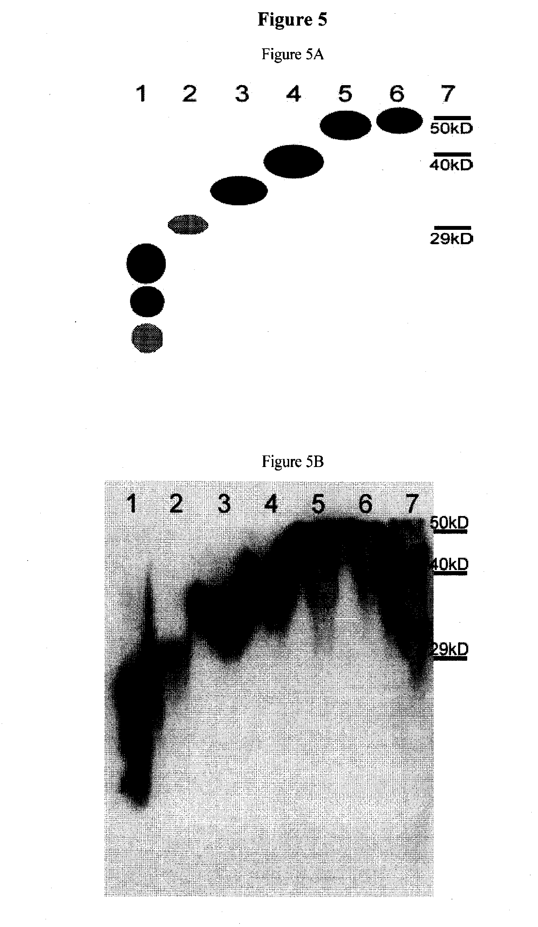

- la figure 5 est une représentation schématique (figure 5A) d'un gel d'électrophorèse (figure 5B) à la suite d'un Western Blot, obtenu après migration d'échantillons de cerveau bovin de BSE traités par des quantités croissantes de triéthylènetétramine (pistes 2-6), la piste 1 correspondant au témoin non traité et la piste 8 correspondant à la piste des marqueurs de poids moléculaire,

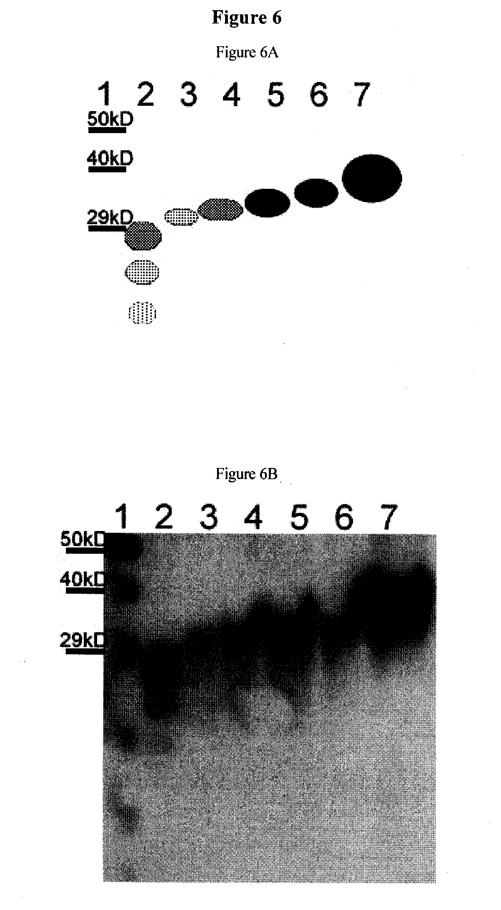

- la figure 6 est une représentation schématique (figure 6A) d'un gel d'électrophorèse (figure 6B) à la suite d'un Western Blot, obtenu après migration d'échantillons de cerveau bovin de BSE traités par des quantités croissantes de bis-3-aminopropylamine (pistes 3-7), la piste 2 correspondant au témoin non traité et la piste 1 correspondant à la piste des marqueurs de poids moléculaire,

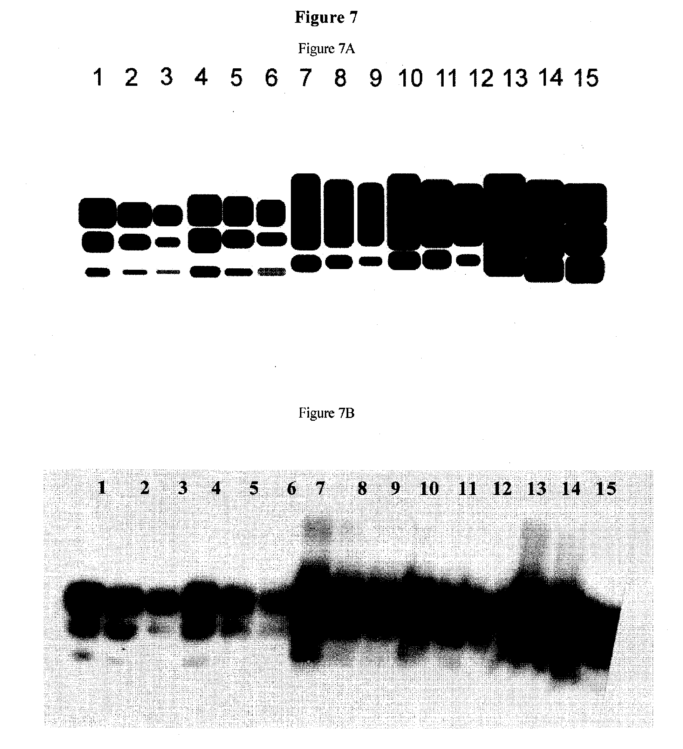

- la figure 7 est une représentation schématique (figure 7A) d'un gel d'électrophorèse (figure 7B) à la suite d'un Western Blot, obtenu après migration d'échantillons de cerveau bovin de BSE traités par de la streptomycine et le calix-arène p-sulfonato-3,7-(2-aminoéthyloxy)-calix-[6]-arène (pistes 7 à 15), les pistes 1 à 3 correspondant aux échantillons témoin sans ajout de streptomycine ni de calix-arène et les pistes 4 à 6 correspondant aux échantillons dans lesquels seul le calix-arène a été ajouté,

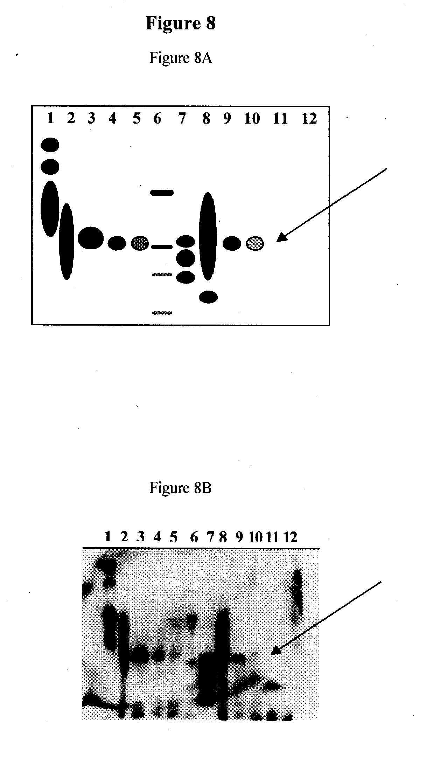

- la figure 8 est une représentation schématique (figure 8A) d'un gel d'électrophorèse (figure 8B) à la suite d'un Western Blot, obtenu après migration d'échantillons de sang humain sain (ou négatif pour la maladie de Creutzfeld-Jakob) surchargé avec 1% d'homogénat de rate, positif ou négatif pour la maladie de Creutzfeld-Jakob (respectivement MCJ+ ou MCJ-), traités par de la streptomycine et de l'héparine (pistes 1 à 5 pour MCJ+ et pistes 8 à 12 pour MCJ-), la piste 6 correspondant à la piste des marqueurs de poids moléculaire et la piste 7 correspondant à l'échantillon témoin positif,

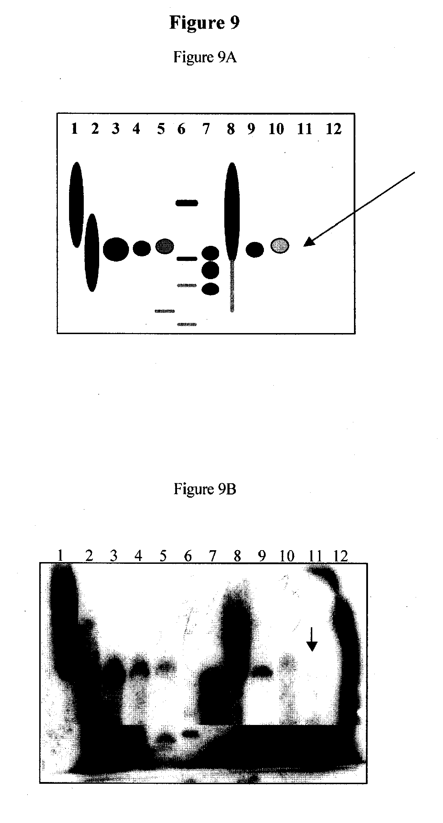

- la figure 9 est une représentation schématique (figure 9A) d'un gel d'électrophorèse (figure 9B) à la suite d'un Western Blot, obtenu après migration d'échantillons de sang humain sain surchargé avec 5% d'homogénat de rate, positif ou négatif pour la maladie de Creutzfeld-Jakob (respectivement MCJ+ ou MCJ-), traités par de la streptomycine et de l'héparine (pistes 1 à 5 pour MCJ+ et pistes 8 à 12 pour MCJ-), la piste 6 correspondant à la piste des marqueurs de poids moléculaire et la piste 7 correspondant à l'échantillon témoin positif, et

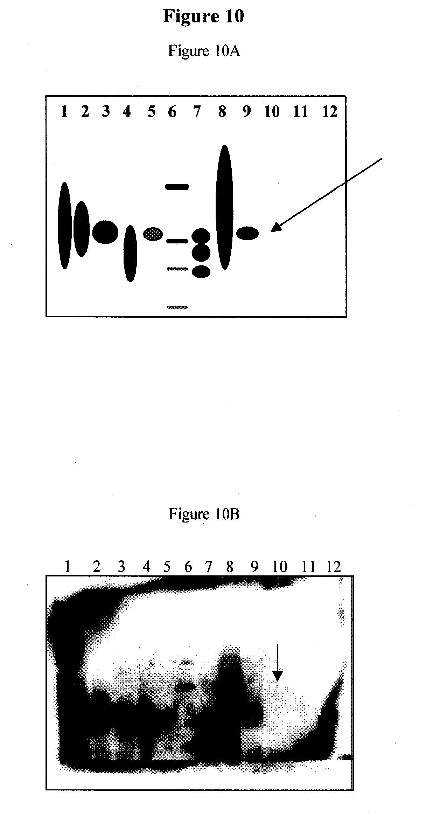

- la figure 10 est une représentation schématique (figure 10A) d'un gel d'électrophorèse (figure 10B) à la suite d'un Western Blot, obtenu après migration d'échantillons de sang humain sain surchargé avec 10% d'homogénat de rate, positif ou négatif pour la maladie de Creutzfeld-Jakob (respectivement MCJ+ ou MCJ-), traités par de la streptomycine et de l'héparine (pistes 1 à 5 pour MCJ+ et pistes 8 à 12 pour MCJ-), la piste 6 correspondant à la piste des marqueurs de poids moléculaire et la piste 7 correspondant à l'échantillon témoin positif.

### Exemple 1 : prétraitement des échantillons

### 1. Echantillons d'organes solides tels qu'extrait de cerveau, non traités à la streptomycine

Les organes (cerveau, etc.) sont tout d'abord homogénéisés dans une solution de glucose à 5% (p/v) afin d'obtenir une suspension à 10%.

A 100 µL de la solution homogénéisée décrite ci-dessus sont ajoutés 5 µL d'une solution de la protéinase K (PK) dosée à 2mg/L, 1 µL d'une solution de SDS (Sodium Dodécyl Sulfate) à 10% et 6 µL d'une solution de N-octyl-β-_{D}-glyco-pyranoside à 2.5% p/v dans l'eau.

Le mélange est agité au vortex puis incubé à 37°C pendant 60 minutes.

500 µL d'un mélange chloroforme : méthanol (1 : 2) sont ensuite ajoutés et le mélange est soumis à une agitation au vortex.

Ceci est suivi d'une étape de centrifugation à 15000tr/min à température ambiante pendant 10 minutes

Le surnageant est alors éliminé et le culot est remis en suspension dans 25 µL d'une solution d'hydrochlorure de guanidine à 6M. La suspension est incubée à 37°C pendant 30 minutes.

Enfin, 400 µL d'une solution de PBS (Phosphate Buffer Saline) et du Tween 20 (0.05% p/v) sont ajoutés.

### 2. Echantillons d'organes solides tels qu'extrait de cerveau, traités à la streptomycine

Les organes (cerveau, rate etc.) sont tout d'abord homogénéisés dans une solution de glucose à 5% (p/v) afin d'obtenir une suspension à 10%.

A 100 µL de la solution homogénéisée décrite ci-dessus sont ajoutés 5 µL d'une solution de la protéinase K (PK) dosée à 2mg/L et 20 µL d'une solution de N-octyl-β-_{D}-glyco-pyranoside à 2.5% p/v dans l'eau. Le mélange est agité au vortex puis incubé à 37°C pendant 30 minutes.

20 µL d'une solution de sulfate de streptomycine à une concentration de 1g/mL sont ajoutés, le mélange est placé sous agitation et re-incubé à 37°C pendant 1 heure.

500 µL d'un mélange chloroforme : méthanol (1 : 2) sont ensuite ajoutés et le mélange est soumis à une agitation au vortex.

Ceci est suivi d'une étape de centrifugation à 10 000 tr/min à température ambiante pendant 10 minutes.

Le surnageant est alors éliminé et le culot est remis en suspension dans 25 µL d'une solution d'hydrochlorure de guanidine à 6M. La suspension est incubée à 37°C pendant 30 minutes.

Enfin, 400 µL d'une solution de PBS et du Tween 20 (0.05% p/v) sont ajoutés.

### 3. Echantillons de fluides biologiques tels que sérum et plasma, traités à la streptomycine

Une solution de protéinase K est ajoutée à 100 µL de sérum ou plasma de sorte que la concentration finale en enzyme soit de 80 µg/mL. La solution est ensuite mélangée au vortex et incubée à 37°C pendant 30 minutes.

20 µL d'une solution de sulfate de streptomycine à une concentration de 1g/mL sont ajoutés, le mélange est placé sous agitation et re-incubé à 37°C pendant 1 heure.

Après centrifugation à 15000tr/min pendant 10 minutes, le surnageant est rejeté.

Le culot résiduel est remis en suspension dans 25 µL d'une solution d'hydrochlorure de guanidine à 6M dans l'eau à l'aide d'une agitation au vortex. La suspension est ensuite incubée à 37°C pendant 30 minutes.

Puis sont ajoutés 400 µL d'une solution de PBS avec 0,05% (p/v) de tampon Tween 20, le mélange est agité au vortex.

### 4. Echantillon de fibrilles (SAF) de PrP^{sc} digérées (PrP^{res}) purifiées, préparé à partir du système nerveux

Les organes (cerveau, etc.) sont tout d'abord homogénéisés dans une solution de glucose à 5% (p/v) afin d'obtenir une suspension à 10% et filtrés.

La PK est utilisée à raison de 25 µg pour 100 mg de tissu. Après homogénéisation au vortex, le tube est placé 1 heure à l'étuve à 37°C.

L'action de la PK est stoppée par homogénéisation avec du Péfabloc® à 10 mM.

La préparation de l'ultracentrifugation comporte l'homogénéisation de 1000 µL de broyat digéré avec 600 µL de Sarkosyl à 30%. Le mélange est laissé au moins 15 minutes à température ambiante avant d'être transféré sur coussin de saccharose (400 µL de saccharose 10%) dans des tubes pour ultracentrifugation (Beckman, Quick Seal, 2,2 ml). Le tube est complété avec de l'eau ultra pure, puis soudé et placé dans le rotor pour ultracentrifugation.

Celle-ci dure 2 heures à 100 000 tr/min. et 20°C.

Les 2/3 du surnageant, dont la fraction lipidique, sont ensuite aspirés. Le culot est séché par retournement sur papier absorbant.

Puis le culot est repris par 100 ou 200 µL de PBS pour une utilisation immédiate ou de tampon de dénaturation pour congélation. Dans ce dernier cas, les culots sont chauffés pendant 10 minutes à 100°C. Après refroidissement, les tubes sont centrifugés pendant 5 minutes à 12000 tr/min. à 20°C. Les surnageants sont congelés à -80°C.

Ces échantillons servent de standards « positifs » de PrP^{res} purifiée.

### Exemple 2: Préparation du ligand calix-arène p-sulphonato-3,7-(2-amino-éthyloxy)-calix-[6]-arène (dénommé C6S)

### 1. Préparation

Ce ligand adjuvant macrocyclique C6S possède la formule générale :

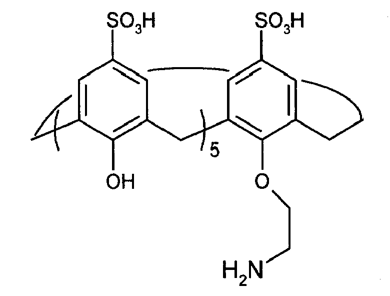

Ce ligand est préparé selon la méthode décrite dans Eric Da Silva et Anthony W. Coleman, Synthesis and complexation properties towards amino acids of mono-substituted p-sulphonato-calix-[n]-arene, Tetrahedron 59 (2003) 7357-7364.

Ce ligand peut ensuite être couplé avec un support solide (bille ou plaque) portant une surface activée comme indiqué ci-après.

### 2. Greffage du ligand C6S sur plaques

Les plaques « NHS activated plates » de 96 puits proviennent de la société Covalab (Lyon, France). 100 µL d'une solution de ligand sont dissous à différentes concentrations dans du Tampon Phosphate 50mM pH 8,2. Les puits sont lavés 3 fois (3 x 200 µL) à l'eau MilliQ après deux heures d'incubation à 37°C. Les plaques sont séchées à température ambiante avant leur utilisation.
Schéma de greffage :

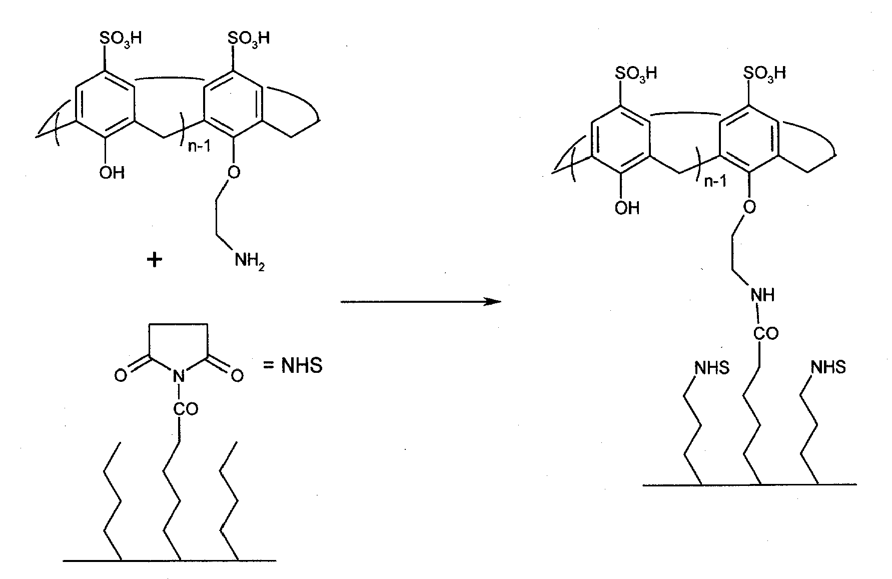

### 3. Greffage sur billes :

4 mL d'une solution de bille activée NHS (2 x 10⁹ billes /mL ; Dynabeads® M270Amine, Société Dynals, Norvège) sont aliquotés en tubes de 1 mL. Les billes sont centrifugées et précipitées par aimantation. Le surnageant est enlevé et les billes sont lavées 3 fois avec 1 mL d'eau. Le culot de bille est repris avec différents volumes de solution de calixarène dans un tampon phosphate 50mM pH 8,2. 600 µL, 120 µL, 60 µL et 12 µL d'une solution de ligand à 50 mg/mL (tampon phosphate 50mM, pH 8,2) sont ajoutés. Les billes sont agitées pendant 24 heures à température ambiante. Les billes sont lavées 3 fois avec de l'eau MilliQ 18Ω afin d'éliminer le ligand qui n'a pas réagi. Les billes sont conservés dans 1 mL d'eau afin de reconstituer la concentration initiale de 2 x 10⁹ billes / mL. La solution de bille est prête à l'emploi. Ces billes sont définies comme « billes C6S » dans tout le texte.
Schéma de greffage du C6S sur les billes :

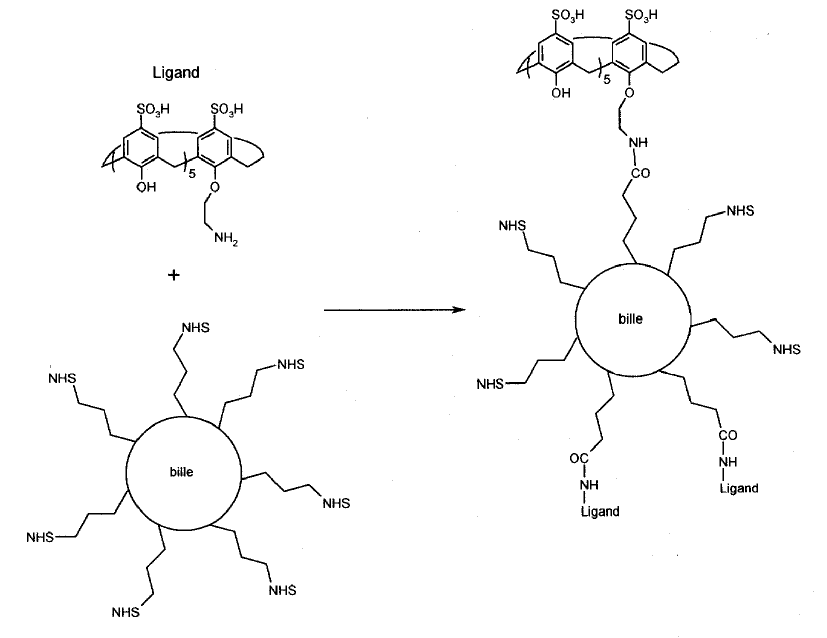

### Exemple 3 : Détection de la PrP^{res}

### 1. Echantillons utilisés

Les échantillons de cerveaux, sérum, plasma bovins positifs sont issus d'animaux dont l'atteinte par une maladie à prion EST (Encéphalite Spongiforme Transmissible) a été confirmée par les méthodes classiques de référence dont une recherche par Western blot de PrP^{res} dans le tissu cérébral comme décrite par Madec et al, 2000, Microbiol pathogenesis, 28 : 353-362. Les témoins négatifs correspondent à des échantillons de cerveaux, sérum et plasma d'animaux pour lesquels l'atteinte par les maladies à prion a été exclue par les mêmes analyses. L'anti-coagulant utilisé pour les prélèvements de plasma est l'EDTA.

Ces échantillons sont fournis par l'AFSSA (Agence Française de Sécurité Sanitaire des Aliments) Lyon FRANCE.

### 2. Détection de la PrP^{res} selon une méthode de type ELISA

Avant utilisation, les puits des plaques obtenues selon l'exemple 2, point 2 ci-dessus sont pré-saturées par un traitement par un mélange lait écrémé (5%) et PBS/Tween 20 (0.05% p/v) à 37°C pendant 60 minutes. Les puits sont ensuite lavés 3 fois avec 800 µL de PBS/Tween 20 (0.05% p/v) par puit. Le tampon résiduel est éliminé par retournement.

100 µL de chaque échantillon de fluide biologique préparé selon l'exemple 1, point 3 ci-dessus, sont distribués dans les puits et la plaque est incubée à 37°C pendant 100 minutes sous agitation mécanique à 400 tr/min.

Suite à cette première incubation, chaque puit est relavé trois fois avec 800 µL du mélange PBS / Tween 20 (0.05% p/v) puis les plaques sont séchées.

L'anticorps anti-PrP de révélation, marqué par à la peroxydase et dilué à 0.05µg/mL dans le mélange PBS / Tween 20 (0.05% p/v) est ajouté à raison de 100 µL par puit et la plaque est incubée de nouveau à 37°C pendant 60 minutes. L'anticorps utilisé reconnaît la région définie par les acides aminés 145-154 de la PrP humaine et les régions homologues des PrP animales (anticorps AC23).

La plaque est alors rincée trois fois avec 800 µL d'une solution de PBS/Tween 20 (0.05% p/v) (PW41, Sanofi Pasteur) et le tampon résiduel est éliminé par retournement.

La révélation est effectuée par ajout de 100 µL dans chaque puit, d'une solution de révélation préparée selon les recommandations du fabricant (kit bioMérieux). La plaque est incubée à température ambiante dans le noir pendant 10 minutes.

La réaction est stoppée par ajout de 50 µL d'une solution d'acide sulfurique (1.8N).

La lecture du signal obtenu est effectuée à l'aide d'un spectrophotomètre à une longueur d'onde de 490nm (Spectrophotomètre PR2100, Biorad).

### 3. Détection de la PrP^{res} dans le cerveau selon la technique Western Blot

Le protocole utilisé correspond au protocole de référence utilisé pour le diagnostic de certitude des maladies à prion chez l'animal et décrit par Madec et al, 2000, Microbiol pathogenesis, 28 : 353-362.

### 4. Résultats

Les résultats sont présentés dans le tableau 1 suivant :
Tableau 1 : Représentation de la spécificité de détection par la méthodologie de type ELISA précédemment décrite de la PrP^{res} dans le sérum et le plasma de bovins par comparaison avec le protocole de référence de type Western Blot effectué à partir du cerveau des mêmes animaux.

**Tableau 1**

| | | **ELISA** | | | |
|---|---|---|---|---|---|
| | | ***Sérum*** | | ***Plasma*** | |
| | | Positif | Négatif | Positif | Négatif |
| **Western** **Blot** ***Cerveaux*** | Positif | **6** | **0** | **3** | **0** |
| | Négatif | **0** | **8** | **0** | **1** |

Ces résultats montrent bien que le procédé de l'invention permet de détecter la PrP dans les fluides biologiques en raison de la concordance entre les deux tests (procédé de l'invention et Western Blot de référence).

Par ailleurs, le procédé de l'invention est également applicable aux fluides physiologiques tels que les sérums et plasmas.

### Exemple 4 : Application du procédé de l'invention aux différentes espèces

### 1. Echantillons

Outre les échantillons utilisés dans l'exemple 3 ci-dessus, on a utilisé des échantillons de cerveaux, sérum, plasma ovins positifs issus d'animaux dont l'atteinte par la maladie à prions scrapie (tremblante) a été confirmée par une recherche par Western blot de PrPres dans le tissu cérébral, ainsi que des plasmas d'ovins infectés par une souche d'ESB provenant de cerveaux bovins (comptabilisés comme bovin positif).

Les échantillons de sérum murin ont été prélevés sur des souris C57BL6 auxquelles ont été préalablement inoculés par injection intra-péritonéale, 100 µL d'une suspension de cerveau de mouton atteint par une souche de scrapie C506M3 adaptée à la souris, à 10% dans une solution de glucose 5% et diluée au 1/200^{ème}. Le sang a été collecté au niveau du sinus orbital 15 jours après l'inoculation.

Ces échantillons ont également été fournis par l'AFSSA (Agence Française de Sécurité Sanitaire des Aliments) Lyon FRANCE.

### 2. Détection de la PrP^{res} dans les fluides biologiques selon une méthode de type ELISA

On a repris le protocole décrit dans l'exemple 3, point 2 ci-dessus.

### 3. Résultats

Les résultats sont reportés sur la figure 1 donnant les valeurs de DO obtenues dans des échantillons de sérum de souris, de sérums d'ovins et de plasmas de bovins, positifs ou négatifs (+ ou -).

Cette figure met en évidence que, de façon surprenante, quelle que soit l'espèce considérée, la valeur de densité optique des échantillons issus d'animaux atteints de maladie à prions est significativement positive comparée aux échantillons issus d'animaux témoins.

Le procédé de l'invention est donc applicable à différentes espèces.

### Exemple 5 : Utilisation de différents anticorps dans le procédé de l'invention

### 1. Echantillons

On a utilisé des échantillons de bovin comme décrit dans l'exemple 3 ci-dessus.

### 2. Détection de la PrP^{res} dans les fluides biologiques selon une méthode de type ELISA

On a repris le protocole décrit dans l'exemple 3, point 2 ci-dessus en utilisant comme anticorps de révélation, soit l'anticorps AC23 tel que décrit précédemment, soit l'anticorps 8D11G12 (bioMérieux, France).

### 3. Résultats

Les résultats sont reportés sur la figure 2 donnant les valeurs de DO obtenues dans des échantillons de plasmas de bovins positifs ou négatifs (+ ou -).

Cette figure met en évidence que le procédé de l'invention peut être mis en oeuvre avec différents anticorps anti-PrP.

### Exemple 6: Détection de la PrP^{res} dans les organes solides

### 1. Echantillons

Les échantillons utilisés correspondent :
i) à des échantillons de cerveau issus de bovins positifs et négatifs dont l'atteinte par une maladie à prion à été confirmée ou infirmée par les méthodes classiques de référence dont la recherche par Western Blot de PrPres dans le tissu cérébral et
ii) à des cerveaux et rates prélevés sur des souris ayant déjà été inoculées par voie intracérébrale (I/C) avec une souche de scrapie adaptée aux souris et montrant des symptômes spécifiques de la maladie.

Ces échantillons ont été fournis par l'AFSSA (Agence Française de Sécurité Sanitaire des Aliments) Lyon FRANCE.

Ils ont été traités comme indiqué dans l'exemple 1, point 2 ci-dessus.

### 2. Détection de la PrP^{res} dans les organes solides selon une méthode de type ELISA

On a repris le protocole décrit dans l'exemple 3, point 2 ci-dessus.

### 3. Résultats

On a comparé les résultats obtenus sur les mêmes échantillons d'organe solide par la méthode de référence Western Blot avec ceux obtenus par le procédé de l'invention.

Les résultats mettent en évidence que l'utilisation du procédé de l'invention combinant à la fois l'utilisation de streptomycine lors de préparation des cerveaux et l'utilisation d'un calix-arène selon un procédé de type ELISA a permis la détection de PrP^{res} dans tous les échantillons positifs.

Cette utilisation du procédé de l'invention permet également la détection de PrP^{res} dans les cerveaux et rates prélevés de souris inoculées par une souche de scrapie adaptée aux souris.

### Exemple 7: Détection de la PrP^{res} dans le sérum et le plasma humain

### 1. Echantillons

Les échantillons utilisés correspondent à des échantillons de plasma et de sérum issus de patients positifs et négatifs dont l'atteinte par la maladie de Creutzfedt-Jakob a été confirmée ou infirmée par les méthodes classiques de référence.

### 2. Détection de la PrP^{res} dans le sérum et le plasma selon une méthode de type ELISA

On a repris le protocole décrit dans l'exemple 3, point 2 ci-dessus en utilisant comme anticorps de révélation, soit l'anticorps AC23 tel que décrit précédemment, soit l'anticorps 8D11G12 (bioMérieux, France).

### 3. Résultats

Les résultats sont indiqués sur la figure 3 qui est une représentation graphique donnant les valeurs de DO obtenues après détection par le procédé de l'invention de PrP^{res} dans des échantillons de sérum et de plasmas humains positifs vis-à-vis de la maladie de Creutzfeld-Jakob (MCJ +) ou négatifs (MCJ -) en utilisant deux anticorps de révélation différents.

La figure 3 met en évidence que, de façon surprenante, le procédé de l'invention permet la détection de la PrP^{res} de façon spécifique, dans le sérum et le plasma de patients humain atteints de MCJ.

Le procédé de l'invention est donc également applicable à l'homme.

### Exemple 8: Utilisation de différentes molécules avant au moins une charge positive et/ou au moins une liaison osidique

### 1. Préparation des échantillons

On a préparé l'échantillon à tester à partir d'homogénat de cerveau bovin BSE positif comme suit :
- aux 100 µL d'homogénat à 10% de cerveau bovin BSE positif mis en suspension dans une solution de glucose à 5 %, on a ajouté 1 µg de protéinase K puis on a incubé à 37C° pendant une heure.
- puis on a ajouté 100 µL de tampon Laemmli, on a agité au vortex, on a chauffé 5 min. à 100°C, on a centrifugé à 12000 tr/min. pendant 5 minutes et on a récupéré les surnageants.

On a utilisé des volumes de 5, 6, 8, 10 ou 50 µL de cette suspension, correspondant à 250, 300, 400, 500 ou 2500 µg de tissu de cerveau, pour les expériences décrites ci-dessous, avec ou sans ajout de molécule ayant au moins une charge positive et/ou au moins une liaison osidique.

### 2. Western Blot

Après migration sur un gel d'électrophorèse unidimensionnelle de polyacrylamide 15% en présence de sodium dodécyl sulfate (SDS PAGE) comme décrit par Laemmli, Nature 227 (1970), 680-685, les protéines sont transférées par électrophorèse sur des membranes nitrocelluloses et immunoblottées à température ambiante pendant 60 minutes avec un anticorps monoclonal (Spi-Bio, France) reconnaissant un épitope spécifique de la protéine prion constitué des acides aminés 146-160. L'anticorps secondaire de détection (1/5000) est un anticorps de chèvre dirigé contre les chaînes lourdes et légères des immunoglobulines de souris conjugué à la peroxydase de radis (IgG H+L).

Les blots sont ensuite lavés et les signaux sont détectés par chimiluminescence soit avec un kit ECL (Amersham) sur des films (Biomex light, Kodak) ou avec un super Signal Ultra (Pierce) et visualisation sur Fluor S. Multimager (BioRad)

### 3. Détection de PrP^{sc} en utilisant du sesquisulfate de dihyrdrostreptomycine

Cette molécule est une molécule ayant deux fonctions guanidinium et une fonction ammonium.

On a ajouté, à un volume constant de PrP^{sc} (50 µL) dans l'échantillon préparé dans le point 1 ci-dessus, des concentrations croissantes (0 ; 2,5 ; 5 et 10mg) de sesquisulfate de dihydrostreptomycine ou de Streptomycine à titre de comparaison.

Après incubation à 37°C pendant une heure, on a centrifugé à 12 000 tr/min. pendant 5 minutes et on a récupéré le surnageant (surnageant de départ).

Les culots sont également récupérés et 50 µL d'une solution v/v d'urée 8M et de tampon Laemmli, leur sont rajoutés. Après une vigoureuse agitation sous vortex, ils sont chauffés à 100°C pendant 5 minutes et centrifugés à 12000 tr/min. pendant 5 minutes. Enfin, on a récupéré les surnageants (surnageant de culot).

On a fait migrer les surnageants de départ ainsi que ceux des culots sur SDS PAGE comme indiqué dans le point 2 ci-dessus.

Les résultats de ce test sont indiqués sur la figure 4 donnant une représentation graphique du gel d'électrophorèse obtenu après migration et où les différentes pistes 1 à 15 correspondent aux conditions de traitement données dans le tableau 2 suivant, avec ou sans les molécules à base de streptomycine.

**Tableau 2**

| Piste | Conditions de traitement | Surnageant concerné |
|---|---|---|
| 1 | Sans ; témoin | Surnageant de départ |
| 2 | 2,5 mg de streptomycine | Surnageant de départ |
| 3 | 5 mg de streptomycine | Surnageant de départ |
| 4 | 10 mg de streptomycine | Surnageant de départ |
| 5 | 2,5 mg de sesquisulfate de dihydrostrept. | Surnageant de départ |
| 6 | 5 mg de sesquisulfate de dihydrostrept. | Surnageant de départ |
| 7 | 10 mg de sesquisulfate de dihydrostrept. | Surnageant de départ |
| 8 | Référence de Poids moléculaire | Aucun ; référence |
| 9 | Sans ; témoin | Surnageant de culot |
| 10 | 2,5 mg de streptomycine | Surnageant de culot |
| 11 | 5 mg de streptomycine | Surnageant de culot |
| 12 | 10 mg de streptomycine | Surnageant de culot |
| 13 | 2,5 mg de sesquisulfate de dihydrostrept. | Surnageant de culot |
| 14 | 5 mg de sesquisulfate de dihydrostrept. | Surnageant de culot |
| 15 | 10 mg de sesquisulfate de dihydrostrept. | Surnageant de culot |

Comme indiqué sur la figure 4, en l'absence des molécules testées, toutes les bandes de PrP^{sc} sont identifiées comme étant dans le surnageant et, en présence de Streptomycine ou sesquisulfate de dihydrostreptomycine, le matériel PrP^{sc} est retrouvé dans le culot.

Les résultats montrent que le sesquisulfate de dihydrostreptomycine, comme la streptomycine, ajouté au milieu induit une réticulation de la PrP qui permet sa précipitation complète avec une centrifugeuse simple (pas besoin d'ultracentrifugation). Les agrégats ainsi obtenus peuvent permettre la détection de PrP, le cas échéant après réaction avec un ligand autre qu'un ligand protéique, selon le procédé de l'invention.

### 4. Détection de PrP^{sc} en utilisant du triéthylène tétramine ou TET

Cette molécule est une molécule ayant quatre fonctions ammonium.

Pour cette expérience, on a ajouté des concentrations croissantes de TET (105, 210, 420, 630 et 840µg) à des volumes constants (5 µL) de PrP^{sc} obtenus à partir de 250 µg de cerveau d'un bovin atteint de BSE, préparé selon le point 1 ci-dessus. Le mélange a été immédiatement centrifugé à 12000 tr/min. pendant 5 minutes et le surnageant a été utilisé pour la détection immunologique par Western blot.

Les résultats sont indiqués sur la figure 5 donnant une représentation graphique du gel d'électrophorèse obtenu après migration, où les différentes pistes 1 à 7 correspondent aux conditions de traitement données dans le tableau 3 suivant, avec ou sans TET.

**Tableau 3**

| Piste | Condition de traitement |
|---|---|
| 1 | Sans traitement ; témoin |
| 2 | 105µg de TET |
| 3 | 210µg de TET |
| 4 | 420µg de TET |
| 5 | 630µg de TET |
| 6 | 840µg de TET |
| 7 | Référence de Poids moléculaire |

Les résultats sur la figure 5 montrent que l'accroissement de la quantité de Triéthylène tétramine dans le milieu par ajout de 105, 210, 420, 630 et 840µg, induit spontanément une augmentation de la masse moléculaire apparente de la protéine prion par rapport à un témoin sans molécule. Ainsi, la détection de la protéine prion est proportionnelle à la quantité de Triéthylène tétramine ajoutée. Ces résultats confirment que le TET produit les mêmes effets que la streptomycine sur la PrP, dans les conditions testées, à savoir une réticulation de la PrP proportionnelle à la dose de TET, objectivée par l'accroissement du poids moléculaire apparent des bandes ayant migré dans le gel d'acrylamide.

### 5. Détection de PrP^{sc} en utilisant de la bis-3-aminopropylamine

Cette molécule est une molécule ayant trois fonctions ammoniums.

On a répété le mode opératoire décrit dans le point 3 ci-dessus, à ceci près qu'on a ajouté, à des volumes constants (5 µL) de PrP^{sc} dans l'échantillon préparé dans le point 1 ci-dessus, des quantités croissantes de bis-3-aminopropylamine (130, 260, 520, 780 et 1040µg) et qu'on a incubé 30 min à température ambiante.

Les résultats sont indiqués sur la figure 6 donnant une représentation graphique du gel d'électrophorèse obtenu après migration du surnageant de culot, et où les différentes pistes 1 à 7 correspondent aux conditions de traitement données dans le tableau 4 suivant, avec ou sans bis-3-aminopropylamine.

**Tableau 4**

| Piste | Condition de traitement |
|---|---|
| 1 | Référence de Poids moléculaire |
| 2 | Sans traitement ; témoin |
| 3 | 130µg de bis-3-aminopropylamine |
| 4 | 260µg de bis-3-aminopropylamine |
| 5 | 520µg de bis-3-aminopropylamine |
| 6 | 780µg de bis-3-aminopropylamine |
| 7 | 1040µg de bis-3-aminopropylamine |

Les résultats indiqués sur la figure 6 montrent que l'accroissement de la quantité de bis-aminopropylamine dans le milieu par ajout de 130, 260, 520, 780 et 1040µg, induit une augmentation de la masse moléculaire apparente des 3 bandes de la protéine prion par rapport à un témoin sans molécule. Dans ces conditions, la protéine prion précipite dans les conditions de centrifugation évoquées dans les points précédent, ce qui corrobore aussi des effets analogues à ceux produits par la streptomycine

### 6. Conclusion

Les résultats montrent que les molécules testées ajoutées au milieu induisent une réticulation de la PrP qui permet sa précipitation complète avec une simple centrifugeuse.

Les agrégats ainsi obtenus peuvent permettre la détection de PrP, le cas échéant après réaction avec un ligand autre qu'un ligand protéique, selon le procédé de l'invention.

### Exemple 9: Détection de la PrP^{res} dans le cerveau de bovin par Western Blot par traitement de l'échantillon par le calix-arène C6S et par la streptomycine

Les échantillons utilisés correspondent à des échantillons de cerveaux issus de bovins positifs dont l'atteinte par une maladie à prion a été confirmée par les méthodes classiques de référence dont la recherche par Western Blot de PrP^{res} dans le tissu cérébral.

Les organes sont tout d'abord homogénéisés dans une solution de glucose à 5% (p/v) afin d'obtenir une suspension à 10%.

A 100 µL de la solution homogénéisée décrite ci-dessus est ajouté 1 µL d'une solution de calix-arene C6S à 0,1 M, puis le mélange est agité au vortex et incubé une heure à 37°C.

7 µL d'une solution de la protéinase K (PK) dosée à 2mg/L sont ajoutés et le mélange est agité au vortex puis incubé à 37°C pendant 30 minutes.

20 µL d'une solution de sulfate de streptomycine à une concentration croissante (5%, 10% et 20%) sont ajoutés, le mélange est placé sous agitation et re-incubé à 37°C pendant 1 heure.

Après avoir ajouté 100 µL de tampon Laemmli dénaturant, on chauffe à 100°C pendant 5 min. et on centrifuge à 12000 tr/min. pendant 5 min.. Les surnageants sont rejetés et les culots sont récupérés. On leur ajoute 50 µL d'une solution à 50% v/v d'urée 8M et le tampon de Laemmli.

Après une vigoureuse agitation sous vortex, on chauffe à 100°C pendant 5 min. et on centrifuge à 12000 tr/min. pendant 5 min., puis on récupère les surnageants pour les faire migrer sur SDS-PAGE comme décrit par Laemmli, Nature 227 (1970), 680-685.

Après migration, les protéines sont transférées par électrophorèse sur des membranes de nitrocellulose et immunoblottées à température ambiante pendant 60 min. avec un anticorps monoclonal reconnaissant un épitope spécifique constitué des acides aminés 126-160 (Spi-Bio, France). L'anticorps secondaire (1/5000) est un anticorps de chèvre dirigé contre les chaînes lourdes et légères des immunoglobulines de souris conjuguées à la peroxydase de radis (IgG H+L). Les blots sont ensuite lavés et les signaux sont détectés par chimioluminescence soit avec un kit ECL (Amersham) sur des films (Biomex light, Kodak) ou avec un super Signal Ultra (Pierce) et visualisation sur Fluor S. Multimager (BioRad).

Les résultats sont illustrés par la figure 7 sur laquelle les pistes 1 à 3 correspondent aux échantillons témoin non traités par le calix-arène et la streptomycine, les pistes 4 à 6 correspondent aux échantillons traités au cali-arène seul (traitement selon le mode opératoire ci-dessus, mais sans l'étape de traitement à la streptomycine) et les pistes 7 à 15 correspondent aux échantillons traités au calix-arène, puis à la streptomycine à raison de 5% (pistes 7 à 9), 10% (pistes 10 à 12) et 20% (pistes 13 à 15).

Les résultats mettent en évidence que la détection de PrP^{res} est améliorée par l'utilisation de calix-arène et de streptomycine ensemble et que la sensibilité de ce procédé augmente avec la quantité de la streptomycine utilisée.

### Exemple 10 : Détection de PrP^{res} dans un fluide biologique par Western Blot par traitement de l'échantillon par de l'héparine et par la streptomycine

### 1. Echantillons

### 1.1 : Préparation des extraits de rate pour la réalisation d'un standard positif et négatif

Les extraits de rate ont été préparés à partir de prélèvements autopsiques de rate d'un patient décédé de la maladie de Creutzfeldt-Jakob, type nouveau-variant et d'un patient non atteint par la maladie de Creutzfeldt-Jakob (MCJ).

Les prélèvements de rate ont tout d'abord été homogénéisés dans un tampon de lyse (NP-40 0,5% (Nonediet P-40) + DOC 0,5% (dodésoxycholate de sodium)) afin d'obtenir une suspension à 10%.

Les homogénats ainsi obtenus ont ensuite été centrifugés à 500 g pendant 5 minutes afin d'éliminer les débris cellulaires. Les surnageants ont été conservés pour la surcharge des échantillons de sang.

### 1.2 : Traitement des échantillons sanguins

Un pool de plusieurs sangs, prélevés chez des patients non atteints de la maladie de Creutzfeldt-Jakob (donneurs sains), et mis en présence d'un glycosaminoglycane, en l'occurrence de l'héparine sous la forme de son sel de lithium, est préparé.

### 1.3 : Réalisation et traitement d'échantillons surchargés avec le standard positif et négatif

### 1.3.1 : Surcharge

Le pool de sang, pré-traité avec l'héparine, a été réparti en plusieurs aliquotes de façon à surcharger chaque aliquote avec une concentration différente d'homogénat de rate, positif ou négatif pour la MCJ (1%, 5% et 10%) (v/v), selon les quantités indiquées dans le tableau 5 ci-dessous.

**Tableau 5**

| **Concentration de surcharge** | **Volume d'homogénat ajouté (µl)** | **Volume de plasma (µl)** |
|---|---|---|
| Témoin plasma | 0 | 2150 |
| 1% | 12,5 | 1235 |
| 5% | 62,5 | 1185 |
| 10% | 125 | 1125 |

### 1.3.2 : Digestion par la protéinase K

Après surcharge, les échantillons ont été digérés par la protéinase K (PK) selon une gamme de concentration finale comprise entre 0 et 300 µg/mL (0 - 50 - 100 - 200 et 300 µg/mL dans 250 µL d'échantillon). Après agitation au vortex, les échantillons ont été incubés à l'étuve pendant 30 minutes à 37°C.

### 1.3.3 : Incubation la streptomycine

25 µL de streptomycine (Gibco) dosée à 1g/mL (en eau distillée) ont ensuite été ajoutés au mélange (concentration finale de 200 mg/mL) puis, après homogénéisation au vortex, les échantillons ont été incubés pendant 60 minutes à l'étuve à 37°C.

### 1.3.4 : Préparation pour le western blot

A l'issue de l'incubation, les échantillons ont été centrifugés pendant 10 minutes à 15000 tr/min. et les surnageants ont été éliminés.

Le culot a été remis en solution dans 50 µL de tampon dénaturant SDS et, après chauffage pendant 10 minutes à 100°C, les échantillons ont de nouveau été centrifugés pendant 5 minutes à 12000 tr/min.. On a repris les surnageants pour l'analyse en western blot.

### 2 Détection de la PrP^{res} dans le sang surchargé selon la technique Western Blot

### 2.1 : Migration électrophorétique et transfert

15 µL de chaque surnageant ont été déposés sur gel de bis-tris acrylamide à 12% (NuPage, Invitrogen). La migration se réalise à 200 Volts pendant 45 minutes puis les protéines sont ensuite transférées sur une membrane de PVDF réhydratée par système semi-sec (électrodes en graphite) pendant 1 heure à 21 volts, 110 milliampères et 2 watts.

### 2.2 : Révélation immunologique

La membrane a ensuite été traitée selon les étapes suivantes :
- Saturation de la membrane en PBS-Tween 0,05% (PBST) + Lait 5%
- Incubation 1 nuit à +(2-8)°C
- Rinçage de la membrane en PBST
- Ajout de l'anticorps primaire : 3F4 (Proteogenix, 9620.103), utilisé à 0,2 µg/mL en PBST
- Incubation 1 heure à température ambiante (TA)
- Lavages en PBST
- Ajout de l'anticorps secondaire : Anti-souris couplé à la péroxydase (Jackson, 115-035-062), utilisé dilué au 1/20000 en PBST
- Incubation 30 min. à température ambiante
- Lavages en PBST
- Lavages en PBS pH 7,2
- Imprégnation avec un substrat auto-radiographique (Super Signal, Pierce)
- Développement photographique

### 3. Résultats

Les résultats sont présentés sur les figures 8 à 10, la figure 8 correspondant à la représentation schématique (figure 8A) du gel d'électrophorèse (figure 8B) à la suite du Western Blot obtenu après migration des échantillons sains surchargés avec 1% d'homogénat de rate, MCJ+ ou MCJ-, la figure 9 correspondant à la représentation schématique (figure 9A) du gel d'électrophorèse (figure 9B) à la suite du Western Blot obtenu après migration des échantillons sains surchargés avec 5% d'homogénat de rate MCJ+ ou MCJ-, et la figure 10 correspondant à la représentation schématique (figure 10A) du gel d'électrophorèse (figure 10B) à la suite du Western Blot obtenu après migration des échantillons sains surchargés avec 10% d'homogénat de rate MCJ+ ou MCJ-.

Sur ces gels, les échantillons des pistes 1 à 5 et pistes 8 à 12 ont les caractéristiques indiquées dans le tableau 6 ci-après, la piste 6 correspond à la piste des marqueurs de poids moléculaire et la piste 7 correspond à l'échantillon témoin positif obtenu à partir d'extrait de cerveau de patient décédé de la maladie de Creutzfeld-Jakob, préparé selon la technique de référence utilisée pour le diagnostic de certitude de la MJC en routine (Madec et al., 2000, Microbiol. Pathogenesis, 28 :353-362).

**Tableau 6**

| **Puits** | **Source surcharge** | **PK (µg/mL)** | **Volume dépôt (µL)** |
|---|---|---|---|
| 1 | Rate MJC+ | 0 | 15 |
| 2 | | 50 | |
| 3 | | 100 | |
| 4 | | 200 | |
| 5 | | 300 | |
| 8 | Rate MJC- | 0 | 15 |
| 9 | | 50 | |
| 10 | | 100 | |
| 11 | | 200 | |
| 12 | | 300 | |

Les résultats mettent en évidence une bande résistante à la protéinase K dans l'échantillon de sang surchargé avec de l'homogénat de rate positive pour la MCJ, quelle que soit la densité de surcharge. En effet, cette bande reste visible quelle que soit la concentration en protéinase K pour l'échantillon positif alors qu'elle disparaît lorsque la concentration en PK est supérieure à 100 µg/mL pour l'échantillon négatif (surchargé avec de la rate MCJ(-)).

Ainsi, la réalisation du test avec l'utilisation combinée de l'héparine et de la streptomycine confirme la sensibilité et la spécificité de détection de la PrP^{res} d'origine lymphoïde (rate) dans des échantillons de sang.

Ceci correspond bien à la situation physiopathologique dans laquelle se positionne un test de détection de la PrP^{res} dans le sang d'individus contaminés par le prion pathogène (PrP^{sc}), prélevé avant la phase de neuroinvasion et/ou les premières manifestations cliniques de la maladie.

C'est dans ce contexte qu'un dépistage des dons de sang de personnes contaminées, porteuses saines et/ou en phase sub-clinique, trouve son utilité en santé publique.

## Revendications

1. Utilisation d'une molécule ayant au moins deux fonctions guanidinium et/ou ammonium, à l'exception des antibiotiques, pour la précipitation de la PrP d'un échantillon biologique, issu ou obtenu d'un organisme animal ou humain.

2. Utilisation selon la revendication 1, **caractérisée en ce que** la molécule ayant au moins deux fonctions guanidinium et/ou ammonium est choisie parmi la polyallylamine, le triéthylènetétraamine (TET), la bis-3-aminopropylamine et le tétrachlorhydrate de spermine.

3. Procédé de détection de la PrP, en particulier de la PrP^{sc}, **caractérisé en ce qu'**il comprend l'étape de mise en présence d'un échantillon biologique, issu ou obtenu d'un organisme animal ou humain, avec une molécule ayant au moins deux fonctions guanidinium et/ou ammonium, à l'exception des antibiotiques.

4. Procédé de détection selon la revendication 3, **caractérisé en ce que** la molécule ayant au moins deux fonctions guanidinium et/ou ammonium est choisie parmi la polyallylamine, le triéthylènetétraamine (TET), la bis-3-aminopropylamine et le tétrachlorhydrate de spermine.

5. Procédé de détection selon la revendication 3 ou 4, **caractérisé en ce qu'**il comprend les étapes consistant à :
a) ajouter, à l'échantillon biologique issu ou obtenu d'un organisme animal ou humain, ladite molécule ayant au moins deux fonctions guanidinium et/ou ammonium, à l'exception des antibiotiques,
b) soumettre la solution à un chauffage, puis à une centrifugation et séparer le culot du surnageant,
c) détecter la PrP.

6. Procédé de détection selon l'une quelconque des revendications 3 à 5, **caractérisé en ce qu'**il comprend une étape supplémentaire de dénaturation de l'échantillon.

7. Procédé de détection selon l'une quelconque des revendications 3 à 6, **caractérisé en ce qu'**il comprend une étape supplémentaire d'addition de protéinase K, de préférence précédant l'étape a).

8. Procédé de diagnostic *in vitro* des maladies à prions, **caractérisé en ce qu'**il comprend l'étape de mise en présence d'un échantillon biologique, issu ou obtenu d'un organisme animal ou humain, avec une molécule ayant au moins deux fonctions guanidinium et/ou ammonium, à l'exception des antibiotiques.

9. Procédé de diagnostic selon la revendication 8, **caractérisé en ce que** la molécule ayant au moins deux fonctions guanidinium et/ou ammonium est choisie parmi la polyallylamine, le triéthylènetétraamine (TET), la bis-3-aminopropylamine et le tétrachlorhydrate de spermine.

10. Procédé d'élimination de la PrP d'un échantillon biologique, issu ou obtenu d'un organisme animal ou humain, **caractérisé en ce qu'**il comprend l'étape de mise en présence dudit échantillon biologique avec une molécule ayant au moins deux fonctions guanidinium et/ou ammonium, à l'exception des antibiotiques.

11. Procédé d'élimination selon la revendication 10, **caractérisé en ce que** la molécule ayant au moins deux fonctions guanidinium et/ou ammonium est choisie parmi la polyallylamine, le triéthylènetétraamine (TET), la bis-3-aminopropylamine et le tétrachlorhydrate de spermine.
